# EUROPÄISCHE PATENTSCHRIFT

(11) **EP 1 395 579 B1**
(45) Veröffentlichungstag und Bekanntmachung des Hinweises auf die Patenterteilung: **19.01.2005**
(21) Anmeldenummer: 02740595.0
(22) Anmeldetag: 14.05.2002
(51) Int. Cl.: C07D 405/06

(54) **VERFAHREN ZUR HERSTELLUNG VON TRIAZOLYMETHYLOXIRANEN**
METHOD FOR PRODUCING TRIAZOLYL METHYL OXIRANES
PROCEDE DE PREPARATION DE TRIAZOLYL METHYL OXIRANES

(30) Priorität: 18.05.2001 DE 10124667
(43) Veröffentlichungstag der Anmeldung: 10.03.2004
(73) Patentinhaber: BASF AKTIENGESELLSCHAFT, 67056 Ludwigshafen (DE)
(72) Erfinder: SANDER, Michael, 67117 Limburgerhof (DE); NOACK, Rainer, 04932 Grossthiemig (DE); KAISER, Reinhard, 01984 Meuro (DE)
(86) Internationale Anmeldenummer: PCT/EP2002/005262
(87) Internationale Veröffentlichungsnummer: WO 2002/094819

(56) Entgegenhaltungen:
- EP-A- 0 094 564
- EP-A- 0 330 132
- T. BURGER ET AL: "Synthesis of four 14C-isotopomers of epoxiconazole, a new triazole fungicide" JOURNAL OF LABELLED COMPOUNDS AND RADIOPHARMACEUTICALS, Bd. 38, Nr. 2, 1996, Seiten 173-178, XP008007186 SUSSEX, GB ISSN: 0362-4803
- H. MIYAUCHI ET AL: "Asymmetric synthesis of SM-9164, a biologically active enantiomer of antifungal agent SM-8668" BULLETIN OF THE CHEMICAL SOCIETY OF JAPAN., Bd. 69, Nr. 9, 1996, Seiten 2625-2632, XP002210961 JAPAN PUBLICATIONS TRADING CO. TOKYO., JP ISSN: 0009-2673
- T. KONOSU ET AL: "Payne rearrangement route to the optically active oxirane precursor for the preparation of triazole antifungals" CHEMICAL AND PHARMACEUTICAL BULLETIN., Bd. 40, Nr. 2, 1992, Seiten 562-564, XP002210962 PHARMACEUTICAL SOCIETY OF JAPAN. TOKYO., JP ISSN: 0009-2363
- T. KONOSU ET AL: "Concise synthesis of optically active oxirane precursors for the preparation of triazole antifungals using the Friedel-Crafts reaction of (S)-2-tosyloxypropionyl chloride" TETRAHEDRON LETTERS., Bd. 32, Nr. 51, 1991, Seiten 7545-7548, XP002210963 ELSEVIER SCIENCE PUBLISHERS, AMSTERDAM., NL ISSN: 0040-4039

## Beschreibung

Die vorliegende Erfindung betrifft ein Verfahren zur Herstellung von Triazolylmethyloxiranen der Formel I, 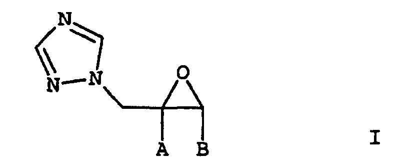 in der A und B gleich oder verschieden sind und unabhängig voneinander C₁-C₄-Alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten ausgewählt aus der Gruppe: Halogen, Nitro, C₁-C₄-Alkyl, C₁-C₄-Alkoxy, Phenoxy, Amino, C₁-C₂-Halogenalkyl oder Phenylsulfonyl tragen kann,
durch Umsetzung eines Oxirans der Formel II, 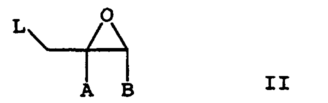 in der A und B die vorgenannte Bedeutung besitzen und L für eine nukleophil substituierbare Abgangsgruppe steht, mit
a) 1,2,4-Triazol (IIIa) und einem Moläquivalent einer anorganischen Base oder
b) einem 1,2,4-Triazolid der Formel IIIb, 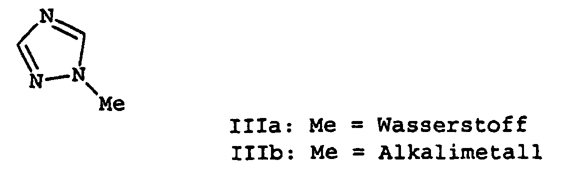 in der Me für ein Alkalimetallatom oder ein quartäres Ammoniumion steht.

Triazolylmethyloxirane dienen zur Herstellung von fungiziden Mitteln, insbesondere gegen Getreidekrankheiten.

Aus EP-A 94 564, US 4,906,652, EP-A 330 132 und EP-A 334 035 sind Verfahren zur Herstellung von Triazolylmethyloxiranen ausgehend von einem Oxiran der Formel II und 1,2,4-Triazol in Gegenwart einer Base bekannt. Die Verfahren werden allesamt bei Raumtemperatur durchgeführt. Die Reaktionszeiten liegen bei 8 bis 18 Stunden. Als Lösungsmittel für die Triazolierung werden im Stand der Technik im allgemeinen dipolare aprotische Lösungsmittel wie Dimethylformamid oder Ether eingesetzt. Im Stand der Technik ist weiterhin nichts bekannt, was auf einen störenden Einfluß von OH-aciden bzw. NH-aciden Verbindungen auf die Regiochemie der Triazolierungsreaktion hindeutet.

Die aus dem Stand der Technik bekannten Verfahren benötigen lange Reaktionszeiten. Durch Solvolyse und Ringöffnungsreaktion entstehen eine Reihe von Nebenprodukten, die die Ausbeute verringern und die Isolierung und Reinigung der gewünschten Triazolylmethyloxirane erheblich erschweren. Weiterhin entstehen neben dem gewünschten 1- auch 4-substituierte Triazole in Anteilen von 5-25%. Bei der üblicherweise durchgeführten Isolierung der Triazolylmethyloxirane durch Ausfällung mit Wasser führen diese Nebenprodukte zu schlechten Zentrifugier- bzw. Filtriereigenschaften der entstehenden Kristallmaischen. In vielen Fällen müssen diese Nebenprodukte vom Wertprodukt durch zusätzliche aufwendige Reinigungsoperationen im nachhinein entfernt werden.

Ohne zusätzliche aufwendigen Reinigungsoperationen lassen sich im Stand der Technik lediglich Reinheitsgrade von unter 92% realisieren. Durch anschließende technische Reinigungsverfahren wie Extraktion oder erneute Fällung kann die Reinheit der fungizid wirksamen Triazolylmethyl-oxirane häufig nur auf 94% gesteigert werden.

Ein wirtschaftliches Verfahren zur Herstellung von Triazolylmethyl-oxiranen auf dem genannten Syntheseweg mit Wirkstoffgehalten von über 96% bei hoher Raum/Zeit-Ausbeute im technischen Maßstab sind bisher nicht verfügbar.

Aufgabe der vorliegenden Erfindung war es demnach ein Verfahren zu finden, daß die obenbeschriebenen Nachteile nicht aufweist und sich im Gegenteil durch eine hohe Prozeßfähigkeit und eine hohe Raum/Zeit Ausbeute auszeichnet. Schließlich sollte das Verfahren Triazolylmethyloxirane in guten Ausbeuten und hoher Reinheit liefern, sodaß aufwendige Reinigungsoperationen für die Endprodukte entfallen können.

Demnach wurde das eingangs erwähnte Verfahren gefunden, das dadurch gekennzeichnet ist, daß man die Triazolierung in einem dipolar aprotischen Lösungsmittel durchführt, dessen Gehalt an OH-aciden Verbindungen weniger als 0,5% und/oder deren Gehalt an NH-aciden Verbindungen weniger als 2,5% beträgt.

In einem Lösungsmittel, das die obengenannten Grenzwerte aufweist, vermindert sich der Gehalt an 4-substituierten Triazolen im Reaktionsprodukt erheblich gegenüber einer Reaktionsführung in Gegenwart OH- und NH-acider Verbindungen gemäß den Verfahren des standes der Technik. Gleichzeitig nimmt der Gehalt an gewünschten 1-substituierten Triazolen zu.

Im folgenden wird das erfindungsgemäße Verfahren näher erläutert.

Als Ausgangsmaterialien eigenen sich Oxirane der Formel II, 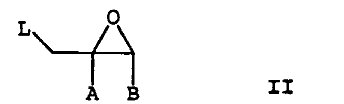 in der A und B gleich oder verschieden sind und unabhängig voneinander C₁-C₄-Alkyl, Phenyl-C₁-C₂-Alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten ausgewählt aus der Gruppe: Halogen, Nitro, C₁-C₄-Alkyl, C₁-C₄-Alkoxy, Phenoxy, Amino, C₁-C₂-Halogenalkyl oder Phenylsulfonyl tragen kann und L für eine nukleophil substituierbare Abgangsgruppe steht. Die Oxirane lassen sich wie in DE-A 39 36823, EP-A 94 564, US 4,906,652, EP-A 330 132 und EP-A 334 035 beschrieben herstellen.

Bevorzugte Ausgangsmaterialien tragen die folgenden Substituenten, wobei die Bevorzugung jeweils für sich allein oder in Kombination zu sehen ist:
A und B bedeuten vorzugsweise einen durch Halogen, C₁-C₄-Alkyl oder C₁-C₄-Alkoxy substituierten Phenylrest.
Insbesondere bedeutet A 4-Fluorphenyl und B 2-Chlorphenyl.
L steht für eine nukleophil substituierbare Abgangsgruppe wie beispielsweise Halogenid, Alkylsulfonat, Arylsulfonat oder Alkylsulfat. Vorzugsweise bedeutet L Chlorid, Bromid, Tosylat und insbesondere Mesylat.

Als Triazolide eignen sich Verbindungen der Formel IIIb, 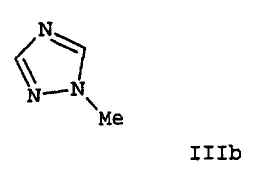 in der Me für ein Alkalimetall oder ein quartäres Ammoniumion wie beispielsweise Tetramethyl-, Tetraethyl-, Tetrabutylammonium steht. Insbesondere steht Me für Natrium oder Kalium.

Geeignete Basen zum Einsatz in Verfahrensvariante a) bzw. zur Herstellung von Triazolid IIIb (Verfahrensvariante b) sind Alkalihydroxide wie beispielsweise Natrium- oder Kaliumhydroxid, quartäre Ammoniumhydroxide, Alkalicarbonate wie beispielsweise Natrium-, Kalium- oder Natriumhydrogencarbonat, Alkalialkoholate wie beispielsweise Natriummethylat oder Kalium-tert.butylat, Alkaliphenolat wie Natriumphenolat, Alkalicarboxylat wie beispielsweise Kaliumformylat oder Natriumacetat oder Hydride wie Natriumhydrid oder Natriumboranat. Für Verfahrensvariante a) eignen sich darüberhinaus Amine wie beispielsweise 4-Dimethylaminopyridin, 4-Pyrrolidinopyridin, Diazabicycloundecen, Iminophosphoranbase oder Phosphazenbase.

Die Durchführung des erfindungsgemäßen Verfahrens erfolgt üblicherweise in Gegenwart eines Lösungs- oder Verdünnungsmittels. Zu den bevorzugten Lösungsmitteln gehören Ketone wie beispielsweise Aceton, Methylethylketon oder Cyclohexanon, Nitrile wie beispielsweise Acetonitril oder Propionitril, Alkohole wie beispielsweise Methanol, Ethanol, iso-Propanol, n-Butanol oder Glykol, Ester wie beispielsweise Essigsäureethylester oder Essigsäurebutylester, Ether wie beispielsweise Tetrahydrofuran, 1,4-Dioxan oder Dimethoxyethan.

Besonders bevorzugte Lösungsmittel sind Amide wie beispielsweise Dimethylformamid, Dimethylacetamid und N-Methylpyrrolidon oder Tetramethylharnstoff.

Als Reaktionsbeschleuniger kommen zusätzlich noch Metallhalogenide wie Natriumiodid, quartäre Ammoniumsalze wie beispielsweise Tetrabutylammoniumchlorid oder Kronenether wie Dibenzo-18-Krone-6 in Betracht. Die Reaktionsbeschleuniger sind jedoch für die Durchführung des Verfahrens nicht notwendig.

Das Verfahren wird in der Regel bei Temperaturen von 10 bis 100°C durchgeführt. Höhere Temperaturen führen zur verstärkten Nebenproduktbildung. Unterhalb von 50°C lassen sich Reaktionszeiten von weniger als 4 Stunden nur unter Inkaufnahme von Ausbeuteverlusten realisieren. Bevorzugt wird in einem Temperaturbereich von 60 bis 70°C gearbeitet.

Die Herstellung der erfindungsgemäßen Triazolidsalzlösung kann auf folgende Weise erfolgen:
1. Lösen eines Triazolidsalzes IIIb in einem Lösungsmittelgemisch von beispielsweise N-Methylpyrrolidon und Toluol und Reduzierung der protischen Verbindungen durch azeotrope Trocknung.
2. Umsetzung eines Triazols IIIa mit einer Base, z.B. Natriummethylat, gelöst in Methanol und Abdestillieren des gesamten Methanols, gegebenenfalls unter Zusatz eines Azeotrope bildenden Lösungsmittels.
3. Lösen eines speziell getrockneten Triazolidsalzes IIIb in einem Lösungsmittel wie Dimethylformamid, Dimethylacetamid, N-Methylpyrrolidon oder Tetramethylharnstoff, wobei deren Gehalt an OH-aciden Verbindungen weniger als 0,5% und/oder deren Gehalt an NH-aciden Verbindungen weniger als 2% beträgt.

Eine spezielle Variante ist die Durchführung der Triazolierungsreaktion unter gleichzeitiger azeotroper Trocknung, beispielsweise durch Zufügen eines Triazols IIIa zu einer toluolischen Lösung von II, Zugabe von methanolischer Lösung einer base, wie Natriummethylat und Abdestillieren der flüchtigen Komponenten bei 60-100°C, gegebenenfalls auch im Vakuum.

Durch das erfindungsgemäße Verfahren wird das Verhältnis von 1-zu 4-substituierten Triazolen (Regioselektivität) auf einen Wert von über 10 angehoben. Der Gehalt an 1-substituiertem Triazol ist in der Regel über 90%. Das erhaltene Produkt ist deshalb wesentlich wirtschaftlicher zu reinigen. Der entsorgungsrelevante Anteil an inaktiven Isomeren und Nebenprodukten sinkt.

Die Raum-Zeit-Ausbeute ist bei dem erfindungsgemäßen Verfahren deutlich höher. Im allgemeinen wird bei einer Reaktionszeit von 2-4 Stunden bei 60 bis 80°C ein nahezu vollständiger Umsatz erzielt.

Das erfindungsgemäße Verfahren wird durch die folgenden Beispiele näher erläutert.

### Herstellungsbeispiele (Synthese des Isomerengemischs Epoxiconazol

### Beispiel 1

In einem 2,5 m³ Reaktor wurden zu 311 kg cis/trans-2-(Methansulfonyloxymethyl)-2-(4-fluorphenyl)-3-(2-chlorphenyl)oxiran (MES/5:95) - gelöst in 1000 kg Toluol -, 675 kg Dimethylformamid, 75 kg Triazol und 187 kg Natriummethanolat (30%-ig in Methanol) hinzugefügt, anschließend wurde auf 65°C erhitzt und unter leichtem Vakuum (ca. 200 mbar) das enthaltene Toluol abdestilliert. Nach dem Toluol und MeOH weitgehend entfernt war, beließ man noch 3h bei 65°C und erhielt nach dem Abkühlen eine Lösung eines Epoxiconazol Wirkstoff-Gemischs mit folgenden analytischen Daten (bezogen auf Feststoff):
trans-Epoxiconazol = 83,5%, sym.-Epoxiconazol = 8,3%, cis-Epoxiconazol = 5,1%;
Damit betrug die Regioselektivität(trans/sym) 10,1.

Durch Fällung und/oder Extraktion konnten die isomeren Epoxiconazole isoliert und anschließend weiter gereinigt werden.

### Beispiel 2

In einem 2,5 m³ Reaktor wurden bei 30°C zu 311 kg cis/trans-2-(Methansulfonyloxymethyl)-2-(4-fluorphenyl)-3-(2-chlorphenyl)oxiran (MES/5:95), gelöst in 675 kg trockenem Dimethylformamid (DMF), 100 kg sprühgetrocknetes Natriumtriazolid mit einem Wassergehalt von 0,2% und einem 1,2,4-Triazolgehalt von 2,5% hinzugefügt und anschließend 5h auf 65°C erhitzt. Man erhielt nach dem Abkühlen eine Lösung von I in Dimethylformamid mit folgenden analytischen Daten (bezogen auf den Feststoff):
trans-Epoxiconazol = 86,5 %, sym.-Epoxiconazol = 7,8 %, cis Epoxiconazol = 5,2, die Regioselektivität betrug 11,1.

### Beispiel 3

In einem 2,5 m³ Reaktor wurden bei 30°C zu 311 kg MES(cis-trans 5:95), gelöst in 500 kg trockenem DMF, zu einer mit Toluol zur Entfernung von Methanol und Wasser azeotrop destillierten Mischung von 75 kg Triazol, 175 kg DMF und 187 kg Natriummethanolat (30%-ig in Methanol) hinzugefügt und anschließend 5h auf 65°C erhitzt. Man erhält nach dem Abkühlen eine Lösung von Epoxiconazol in Dimethylformamid mit folgenden analytischen Daten (bezogen auf Feststoff):
trans-Epoxiconazol = 85,7%, sym.-Epoxiconazol = 7,6%, cis-Epoxiconazol = 5,2%, die Regioselektivität beträgt 11,3.

### Vergleichsbeispiel

Es wurde analog Beispiel 3 verfahren, jedoch auf die azeotrope Destillation der Toluol-Mischung verzichtet. Auf diese Weise wurde ein Produkt mit folgenden analytischen Daten (bezogen auf den Feststoff) erhalten:
trans-Epoxiconazol = 81,3%, sym.-Epoxiconazol = 11,3%, cis-Epoxiconazol = 5,2%, die Regioselektivität betrug 7,2.

## Patentansprüche

1. Verfahren zur Herstellung von Triazolylmethyloxiranen der Formel I, 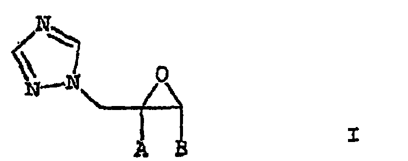 in der A und B gleich oder verschieden sind und unabhängig voneinander C₁-C₄-Alkyl, Phenyl-C₁-C₂-Alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten ausgewählt aus der Gruppe: Halogen, Nitro, C₁-C₄-Alkyl, C₁-C₄-Alkoxy, Phenoxy, Amino, C₁-C₂-Halogenalkyl oder Phenylsulfonyl tragen kann,
durch Umsetzung eines Oxirans der Formel II, 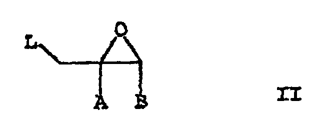 in der A und B die vorgenaante Bedeutung besitzen und L für eine nukleophil substituierbare Abgangsgruppe steht, mit
a) 1,2,4-Triazol IIIa und einem Moläquivalent einer anorganischen Base oder
b) einem 1,2,4-Triazolid der Formel IIIb, 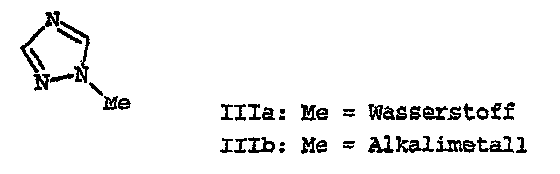 in der Me für ein Alkalimetallatom oder ein quartäres Ammoniumsalz steht, **dadurch gekennzeichnet, daß** man die Triazolierung in einem dipolar aprotischen Lösungsmittel durchführt unter gleichzeitiger azeotroper Trocknung, so dass dessen Gehalt an OH-aciden Verbindungen weniger als 0, 5% und/oder deren Gehalt an NH-aciden Verbindungen weniger als 2,5% beträgt.

2. Verfahren gemäß Anspruch 1, **dadurch gekennzeichnet, dass** man die azeotrope Trocknung mittels Toluol durchführt.

3. Verfahren gemäß einem der Ansprüche 1 bis 2, **dadurch gekennzeichnet, dass** man die anorganische Base unter a) in methanolischer Lösung verwendet.

4. Verfahren gemäß einem der Ansprüche 1 bis 3, **dadurch gekennzeichnet, daß** als dipolar aprotisches Lösungsmittel ein Amid, Harnstoff, Urethan, Sulfoxid oder ein Gemisch dieser Lösungsmittel eingesetzt wird.

5. Verfahren gemäß Anspruch 4, **dadurch gekennzeichnet, daß** als Lösungsmittel Dimethylformamid, Dimethylacetamid oder N-Methylpyrrolidon eingesetzt wird.

6. Verfahren gemäß einem der Ansprüche 1 bis 5, **dadurch gekennzeichnet**, das eine Verbindung mit der Formel I, worin die Substituenten A Fluorphenyl und B chlorphenyl bedeuten, hergestellt wird.

## Claims

1. A method for producing triazolylmethyloxiranes of the formula I, 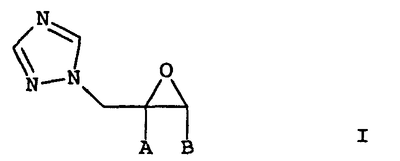 in which A and B are identical or different and, independently of one another, are C₁-C₄-alkyl, phenyl-C₁-C₂-alkyl, C₃-C₆-cycloalkyl, C₃-C₆-cycloalkenyl, tetrahydropyranyl, tetrahydrofuranyl, dioxanyl or phenyl, where the phenyl radical may carry one to three substituents chosen from the group: halogen, nitro, C₁-C₄-alkyl, C₁-C₄-alkoxy, phenoxy, amino, C₁-C₂-haloalkyl or phenylsulfonyl,
by reacting an oxirane of the formula II, 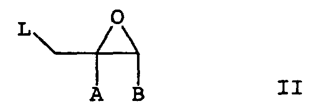 in which A and B have the abovementioned meanings and L is a nucleophilically substitutable leaving group, with
a) 1,2,4-triazole IIIa and one mole equivalent of an inorganic base or
b) a 1,2,4-triazolide of the formula IIIb, 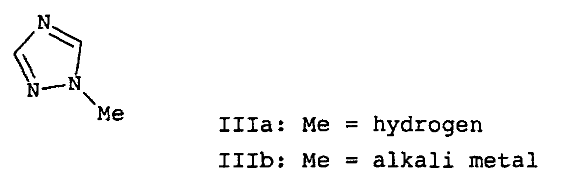
in which Me is an alkali metal atom or a quaternary ammonium ion, which comprises carrying out the triazolation in a dipolar-aprotic solvent with simultaneous azeotropic drying so that its content of OH-acidic compounds is less than 0.5% and/or whose content of NH-acidic compounds is less than 2.5%.

2. A method as claimed in claim 1, wherein the azeotropic drying is carried out using toluene.

3. A method as claimed in either of claims 1 and 2, wherein the inorganic base under a) is used in methanolic solution.

4. A method as claimed in any of claims 1 to 3, wherein the dipolar-aprotic solvent used is an amide, urea, urethane, sulfoxide or a mixture of these solvents.

5. A method as claimed in claim 4, wherein the solvent used is dimethylformamide, dimethylacetamide or N-methylpyrrolidone.

6. A method as claimed in any of claims 1 to 5, wherein a compound with the formula I in which the substituent A is fluorophenyl and B is chlorophenyl is prepared.

## Revendications

1. Procédé de préparation de triazolylméthyloxiranes de la formule I 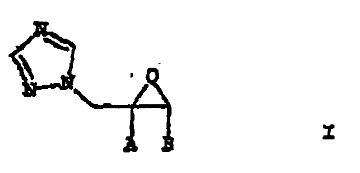 dans laquelle A et B sont identiques ou différents et représentent indépendamment l'un de l'autre des radicaux alkyle en C₁ à C₄, phényl-(C₁-C₂)-alkyle, cycloalkyle en C₃ à C₆, cycloalcényle en C₃ à C₆, tétrahydropyranyle, tétrahydrofuranyle, dioxanyle ou phényle, où le reste phényle peut porter de un à trois substituants choisis dans le groupe formé par les radicaux halogène, nitro, alkyle en C₁ à C₄, alcoxy en C₁ à C₄, phénoxy, amino, halogénoalkyle en C₁ à C₂ ou phénylsulfonyle, par réaction d'un oxirane de la formule II 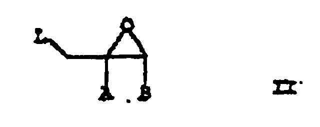 dans laquelle A et B possèdent la signification ci-avant et L représente un groupe partant substituable par voie nucléophile, avec
a) du 1,2,4-triazole IIIa et un équivalent molaire d'une base inorganique ou
b) un 1,2,4-triazolide de la formule IIIb 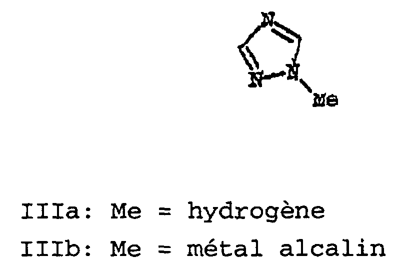
dans laquelle Me représente un atome de métal alcalin ou un sel d'ammonium quaternaire, **caractérisé en ce que** l'on entreprend la triazolation dans un solvant aprotique dipolaire avec séchage simultané azéotropique, de manière que sa teneur en composés à OH acide soit inférieure à 0,5% et/ou que leur teneur en composés à NH acide soit inférieure à 2,5%.

2. Procédé selon la revendication 1, **caractérisé en ce que** l'on entreprend le séchage azéotropique au moyen de toluène.

3. Procédé selon l'une quelconque des revendications 1 à 2, **caractérisé en ce que** l'on utilise la base inorganique sous a) en solution méthanolique.

4. Procédé selon l'une quelconque des revendications 1 à 3, **caractérisé en ce que** l'on met en oeuvre comme solvant aprotique dipolaire un amide, de l'urée, un uréthanne, un sulfoxyde ou un mélange de ces solvants.

5. Procédé selon la revendication 4, **caractérisé en ce que** l'on met en oeuvre comme solvant du diméthylformamide, du diméthylacétamide ou de la N-méthylpyrrolidone.

6. Procédé selon l'une quelconque des revendications 1 à 5, **caractérisé en ce que** l'on prépare un composé de la formule I dans lequel le substituant A est du fluorophényle et le substituant B est du chlorophényle.
